# EUROPEAN PATENT SPECIFICATION

(11) **EP 2 598 479 B1**
(45) Date of publication and mention of the grant of the patent: **01.04.2015**
(21) Application number: 11840625.5
(22) Date of filing: 05.04.2011
(51) Int. Cl.: C07D 209/52, C07C 231/18, C07C 231/20, C07C 233/47

(54) **A METHOD FOR PREPARING RAMIPRIL**
VERFAHREN ZUR HERSTELLUNG VON RAMIPIRIL
PROCÉDÉ DE PRÉPARATION DU RAMIPRIL

(30) Priority: 11.11.2010 IN MU30942010
(43) Date of publication of application: 05.06.2013
(73) Proprietor: Aarti Healthcare Limited, Mumbai 421 204 (IN)
(72) Inventor: DESAI, Parimal, Mumbai 421 204 (IN); SALVI, Narendra, Mumbai 421 204 (IN); PATRAVALE, Bharatkumar, Mumbai 421 204 (IN)
(74) Representative: Ilgart, Jean-Christophe
(86) International application number: PCT/IN2011/000207
(87) International publication number: WO 2012/063252

(56) References cited:
- WO-A1-2009/050041
- WO-A1-2010/049401
- US-A1- 2009 017 509

## Description

### Field of the Invention

The present invention relates to an improved process of preparing Ramipril and to the intermediates used in the process and the use thereof. More particularly, the present invention relates to an improved process for the preparation of an angiotensin-converting enzyme (ACE) inhibitor and further relates to synthesis of desired stereoisomer of an intermediate useful in the preparation of Ramipril.

### Background and prior art

Ramipril, chemically described as [2*S*, 3a*S*, 6a*S*]-1-[(2*S*)-2-[[(1*S*)-1-(Ethoxycarbonyl)-3-phenylpropyl]amino]-1-oxopropyl]octahydrocyclopenta[*b*]pyrrole-2-carboxylic acid, is an ACE inhibitor. Refer to [A] below:

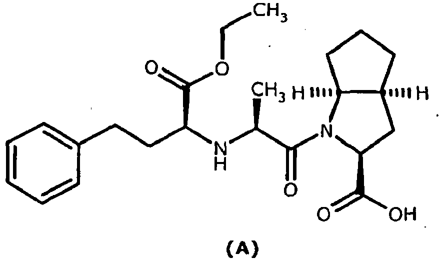

Ramipril is useful to treat hypertension and congestive heart failure. It lowers the production of angiotensin II, therefore relaxing arterial muscles and at the same time enlarging the arteries, allowing the heart to pump blood more easily, and increasing blood flow.

Ramipril and pharmaceutically acceptable salts thereof were first claimed in US4587258. The patent discloses preparation of Ramipril and its intermediates. 2-Azabicyclo [3.3.0]-octane-3-carboxylate (B) and its pharmaceutically acceptable salt is an important intermediate for the synthesis of Ramipril. Intermediate (B) exists in eight stereoisomeric forms. However optically pure cis endo 2-azabicyclo[3.3.0]-octane-3S-carboxylate and its pharmaceutically acceptable salts (Ba) are valuable intermediates for the preparation of Ramipril. Optical purity of Ramipril or its salt is of great importance for the treatment of hypertension, and it depends upon optical purity of (Ba).

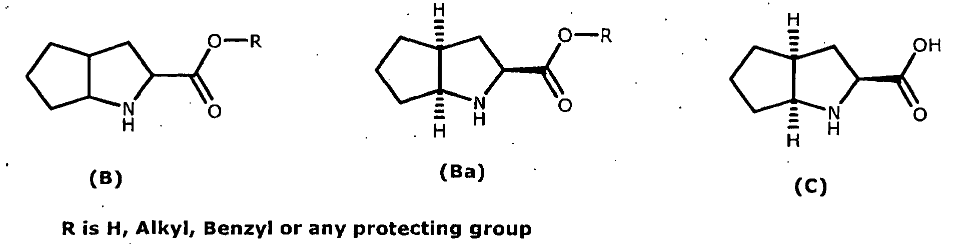

Many attempts have been made to prepare intermediate (B). However there are many difficulties in production of Ba with higher optically purity and excellent yields.

WO2005049568 discloses preparation of racemic compound 7, as shown in the scheme below. The compound is further resolved by diastereomeric salt formation.

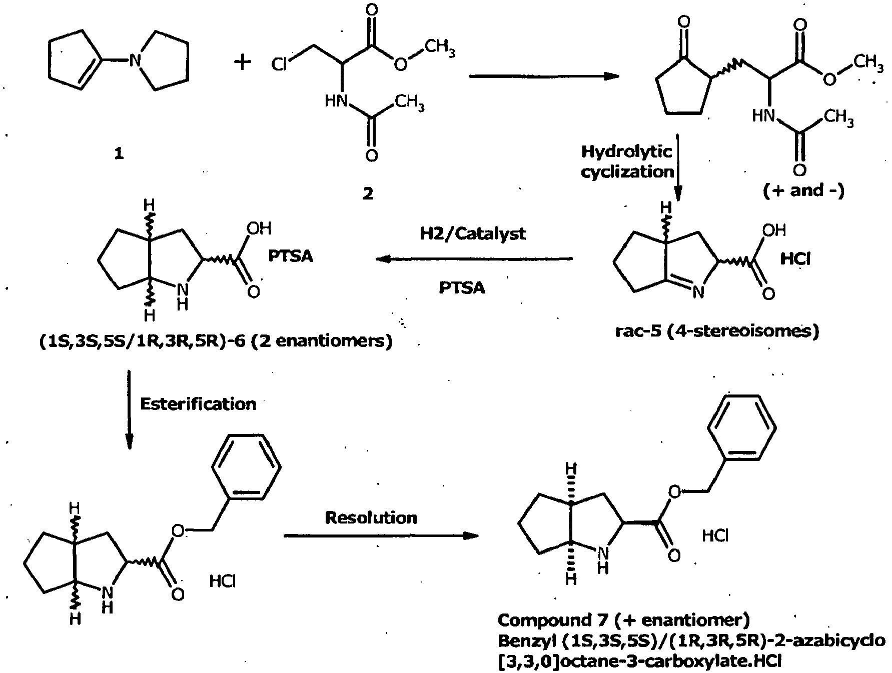

Such a late stage resolution without the possibility of racemising and reusing the unwanted enantiomer leads to inefficient and uneconomical process and thus making it non applicable on industrial scale.

EP0115345B1 describes a method of preparation of Cis, endo benzyl-2-azabicyclo[3.3.0]octane -3-carboxylate hydrochloride by resolving racemic mixture of benzyl-2-azabicyclo[3.3.0]octane-3-carboxylate by crystallization of diastereomeric N-acylated R or S-amino carboxylic acid salts. The process is not cost effective due to usage of relatively commercially less accessible amino acids.

Indian patent application 346/MAS/2001 discloses an improved process for the preparation of optically pure key intermediate IIa for Ramipril, The process comprises reacting racemic (Ba) with alkali hydroxide/alkali carbonate system followed by treatment with L (+) mandelic acid at 0-3°C. Thus the process yields (S,S,S) diasteromeric salt of (Ba). The unwanted enantiomer is waste, although the process gives above 35% yield.

Use of optically active O,O-dibenzoyl-S-tartaric acid for resolving racemic mixtures of imino-α-carboxylic acid esters is claimed in US4659838. However the experimental section shows that the yield of cis, endo isomer does not exceed 85%.

US20070232680 discloses preparation of (S,S,S)-azabicyclo [3.3.0]-octane-3-carboxylic acid benzyl ester hydrochloride having 100% HPLC purity. However the application mentions about HPLC purity and does not mention anything about optical purity. Thus there remains the need for improved process for preparation of intermediate (Ba) with higher optical purity, which can be used further for the preparation of optically pure Ramipril.

Document US2009/0017509 discloses a process for the preparation of a chiral amino acid in two steps. The first one is a simple hydrolysis of an ester with a base/acid to form a racemic amino acid and the second step is the formation of a chiral amino acid from a racemic amidoacid utilising a penicillin G amidase enzyme or vice versa The product is a chiral aminocarboxylic acid. However, no enzymatic breaking of an ester bond is mentioned in this document.

US6407262B1 claims process for separation of diastereoisomeric mixtures of (2S,3aS,6aS)-1-[(S)-2-[[(S)-1-(ethoxycarbonyl)-3-phenylpropyl]amino]propanoyl]octahydrocyclopenta-[b]pyrrole-2-carboxylic acid derivatives and (2R,3aR,6aR)-1-[(S)-2-[[(S)-1-(ethoxycarbonyl)-3-phenylpropyl]amino]propanoyl]-octahydrocyclopenta[b]pyrrole-2-carboxylic acid derivative by dissolving the mixture in a solvent, preferably ethyl acetate, butyl acetate, ethyl propionate, isopropyl acetate, tert-butyl methyl ether, di-isopropyl ether, toluene, dimethoxyethane, acetonitrile, ethanol and mixtures thereof, so as to precipitate desired (S,S,S) isomer. However none of the experiment shows the % yield greater than 31%. This indicates that the process is not feasible on industrial scale.

Synthesis of optically pure (Ba) is also reported in Tetrahedron Lett, 1993, 34 (41), 6603 - 6606 and Heterocycles 1989 28 (2), 957 - 965*,* however the processes results in very low yields of 13% and 5% respectively and hence is not economically feasible for large scale production, which is substantiated by the disclosure of US6407262 (column 3, line 45-65).

WO2010/049401 discloses bioconversion of 3-(2-oxocyclopentyl)-2-uredopropionic acid to (S)-2-amino-3-(2-oxocyclopentyl) propanoic acid using "Expression of Hyu genes in Escherichia Coli RV308)".

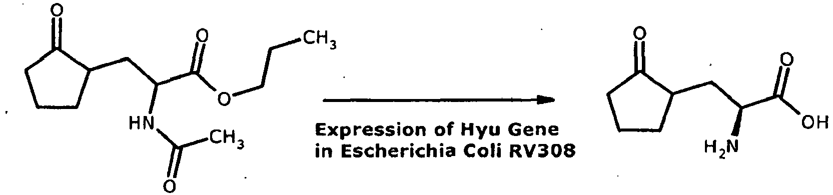

However getting optically pure intermediate (Ba) is very difficult and requires lengthy work up. Moreover the methods give very low yields thus making the process not feasible for industrial application.

Thus there is a need for cost effective process for the preparation of optically pure isomer of key intermediate of formula (Ba), which in turn can yield Ramipril at comparatively low cost.

### Objects of the invention

It is the object of the present invention to provide an improved method of preparing Ramipril with improved purity.

The object of present invention is to provide an improved process for synthesis of desired stereoisomer of an intermediate useful in the preparation of Ramipril.

Another object of present invention is to provide a process for the preparation of Ramipril involving intermediate formed by process of the present invention.

### Detailed description of the invention

In one aspect the present invention provides enantiomer-specific synthesis of optically pure (2S)-acetylamino-3-(2-oxo-cyclopentyl)-propionic acid (1).

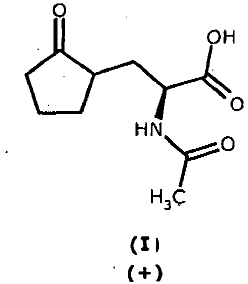

The process can be schematically represented as shown in scheme 1, 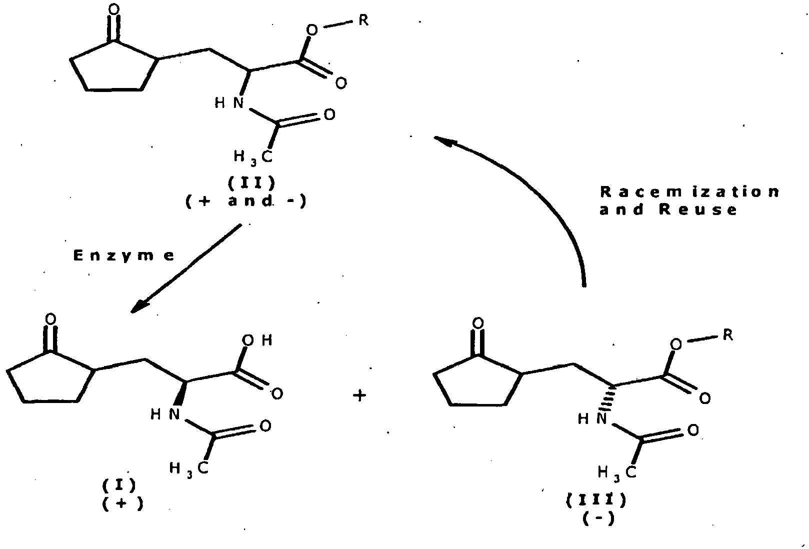 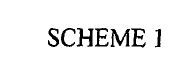 wherein it comprises converting enantiomeric mixture of (1-4C alkyl)-2-(acetylamino)-3-(2-oxocyclopentyl)propanoate (II) (+ and -) under the influence of an enzyme - Alkaline serine endopeptidase , to get (+) isomer of (2S)-acetylamino-3-(2-oxo-cyclopentyl)-propionic acid (I) and (-) isomer (1-4C alkyl) (2R)-2-(acetylamino)-3-(2-oxocyclopentyl)propanoate (III). R is 1-4C alkyl.

Alkaline serine endopeptidase is derived from a selected strain of *Bacillus Licheniformis.* It is widely used in protein processing applications. This enzyme is obtained from *Genencor* as Protex 6L. The enzyme can be employed immobilized or non-immobilized, preferably immobilized. The immobilized enzyme is called as *Immozyme* Alkaline serine endopeptidase. The specification, along with claims, may further contain reference to Protex 6L, which shall be construed to be the said enzyme, i.e. Alkaline serine endopeptidase.

2-Acetylamino-3-(2-oxo-cyclopentyl)-propionic acid ester (II) (+ and -) is dissolved in demineralized water. The solution is purified with activated charcoal. The mixture is filtered and *Immozyme* Alkaline serine endopeptidase, is added to the filtrate. The reaction is carried out at temperature 20-30°C, preferably 23-27°C, most preferably 24-26°C. pH of the solution is adjusted to 6-7, preferably to 6.2 - 6.8 with suitable alkali solution e.g. sodium carbonate and sodium hydroxide preferably sodium carbonate. The alkali solution is prepared by dissolving alkali in pure water. The reaction mixture is maintained under above mentioned conditions for time sufficient to complete the reaction, preferably for around 20 hours. After completion of the reaction the pH of the solution is adjusted to around 6, preferably by using freshly prepared solution of dilute acetic acid. The reaction mixture is filtered to remove *Immozyme Protex 6L.* The enzyme is washed twice with water and this water-wet Immozyme can be directly reused in the next batch. The filtrate is extracted with suitable extracting organic solvent, e.g. dichloromethane, ethylene dichloride, ethyl acetate, chloroform and the like for removal of undesired enantiomer. Preferably the filtrate is extracted with dichloromethane and desired stereoisomer remains in aqueous phase. The undesired (-)-isomer (III) is recovered by concentrating the organic layer and recemized by heating residue with suitable alcoholic solvent e.g. methanol, ethanol, iso-propanol, butanol, preferably methanol. The temperature is raised gradually to reflux. The alcoholic solution is treated with suitable alkali alkoxide, for e.g. sodium methoxide, sodium ethoxide, sodium propoxide, sodium butoxide, potassium methoxide, potassium ethoxide, potassium butoxide and the like, preferably sodium methoxide. The mixture is heated to reflux and maintained at reflux for at least 1 hour. The mass is stirred and recemized II (+ and -) is isolated from organic layer by distillation. Recemized II (+ and -) can be recycled in above step for the preparation of desired (S)-stereoisomer (I). Thus separation and recemization of (III) (-) to (II) (+ and -) and again recycling in the step is achieved to increase overall yield.
The major advantage of this step is undesired (-)-stereoisomer (III) present in the organic layer, can be recemized to compound (II) (+ and -) by simple work up and can be recycled in the step. This avoids wastage of raw materials, which would have been difficult in the later stage. Due to this advantage, the process results, in higher yields and becomes more economical and industrially feasible. Also separated water-wet *Immozyme Protex 6L* can be directly reused in the step with very little loss of activity. For long term storage of *Immozyme Protex 6L,* the used Immozyme is washed thoroughly with cold acetone and dried under air or nitrogen stream. It is stored at around 4°C. Alternatively *Immozyme* is washed with 50% aqueous 1,2-propanediol and stored in this form at -18°C. Before re-use the Immozyme is warmed to 0-5°C and washed thoroughly with water to remove 1,3-propanediol traces. This recovery and re-use of Immozyme Protex 6L further reduces the cost and makes process more economical and feasible, especially for the large scale preparation.

The aqueous layer is purified by stirring continuously with activated charcoal for at least 15 minutes. The layer is filtered. The pH is adjusted to 3 by using suitable resin preferably Amberlyst 15. The reaction mass is filtered and filtrate is evaporated under reduced pressure to get optically pure isomer (I). Use of mineral acids for pH adjustment is avoided because of formation of inorganic salts in the evaporated product (I) which complicates the isolation step. Amberlyst 15 is strongly acidic, macroreticular polymer resin having sulfonic acid groups used in both aqueous and non-aqueous systems. Isolation of pure isomer (I) is achieved by using Amberlyst 15, as it does not add substantial acidic species into the streams in contact with it. The resin is activated prior to use by stirring it with concentrated hydrochloric acid. Optically pure (2S)-acetylamino-3-(2-oxo-cyclopentyl)-propionic acid (I) thus obtained is optionally purified by heating with butanone to get clear solution. The solution is cooled gradually to get thick crystal slurry, which is filtered and washed with cold butanone.

In another aspect present invention provides synthesis of key intermediate (Ba) by hydrolytic cyclization of isolated (S)-stereoisomer (1) obtained by the process of the present invention. The process can be schematically represented as shown below in Scheme 2.

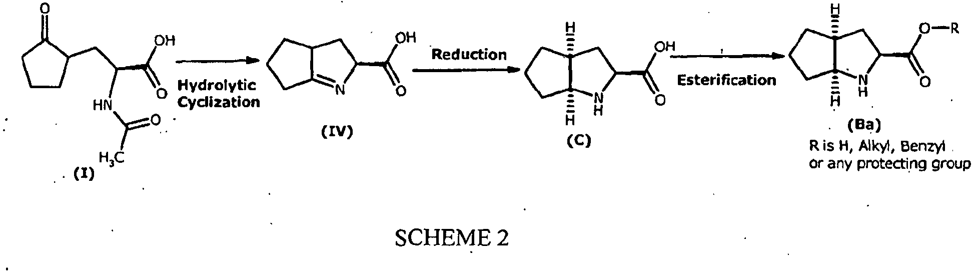

The reaction results the formation of (2S)-2,3,3a,4,5,6-Hexahydro-cyclopenta[b]pyrrole-2-carboxylic acid (IV). Hydrolytic cyclization is carried out by refluxing (I) in presence of suitable cyclizing agent for e.g. concentrated hydrochloric acid, sulfuric acid, para-toluene sulfonic acid and methane sulfonic acid. Thus cyclized product (IV) is isolated preferably in the form of acid addition salt. For example as an acid salt of hydrochloric acid, sulfuric acid, para-toluene sulfonic acid and methane sulfonic acid. The isolated salt of (IV) is hydrogenated with suitable hydrogenating agents to yield (2S,3aS,6aS)-octahydrocyclopenta [*b*]pyrrole-2-carboxylic acid (C) which can also be optionally isolated as acid addition salt for e.g. hydrochloric acid, sulfuric acid, para-toluene sulfonic acid and methane sulfonic acid. The hydrogenation is carried out using conventional methods, preferably catalytic hydrogenation in presence of catalysts palladium, platinum. Optionally compound (C) is converted to an ester (Ba) e.g. alkyl, benzyl or any other suitable protecting group. The ester can also be isolated as acid salt e.g. hydrochloric acid, sulfuric acids, para-toluene sulfonic acid and methane sulfonic acid. In another aspect the invention provides a process for the preparation of Ramipril, using intermediate (Ba) prepared by the process of the present invention as stated in scheme 2 above. The process comprises, reacting intermediate (Ba), with 2-(4-methyl-2,5-dioxo-oxazolidin-3-yl)-4-phenyl-butyric acid ethyl ester of formula (C), to give Ramipril

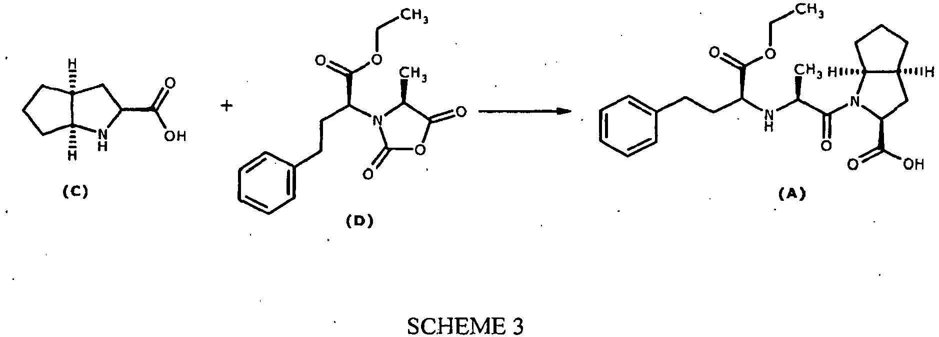

Intermediate (C) prepared by the process of the present invention is condensed with ethyl (2S)- [(4S)- methyl-2,5-dioxo-1,3-oxazolidin-3-yl]-4-phenylbutanoate (C), in a suitable solvent e.g. dichloromethane or water at pH 8-12 preferably at pH 10.5 at temperature 20-25°C to yield optically pure Ramipril (A).

In still another object preparation of Ramipril is achieved by condensing suitable ester e.g. alkyl, benzyl or any other suitable protecting group of hydrochloride (Ba) formed by the process of the present invention with (2S)-2-([(2S)-1-ethoxy-1-oxo-4-phenylbutan-2-yl]amino) propanoic acid to form alkylated or benzylated Ramipril.

This can be schematically represented as shown in Scheme 4.

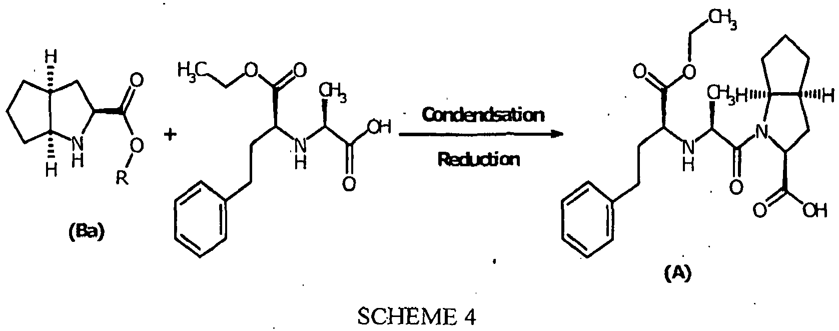

The condensation is carried out in presence of suitable condensing agent preferably in presence of 1-hydroxy benzotriazole (HOBt). The solvent used in the process is high broiling solvent preferably toluene. The reaction is carried out at 0-5°C. Ramipril ester thus obtained is deprotected by reduction with suitable hydrogenating agent preferably catalytic hydrogenation in presence of Pd/C, to form Ramipril (A). The present invention is further illustrated by following exemplary embodiments, which should not be construed as limiting the scope of the invention.

### EXAMPLES

### Example 1

### Preparation of (2S)-Acetylamino-3-(2-oxo-cyclopentyl)-propionic acid (I)

Methyl 2-(acetylamino)-3-(2-oxocyclopentyl)-propionate (90 gm) was dissolved in water (350 ml) with constant stirring to get a clear solution. pH of the solution was adjusted to 6.8 by freshly prepared solution of sodium carbonae. *Immozyme protex 6L* (0.75 g) was added to the solution and with constant stirring, pH of the reaction mass was adjusted to 6.2 - 6.8 at 24 - 26°C by sodium carbonate solution. Reaction mass was maintained for 20 hours. pH of the solution was adjusted to 6 by freshly prepared 10% Acetic acid solution. The reaction mass filtered to separate catalyst and filtrate was extracted with MDC (3 X 250 ml). After separation of both the phases, pH of the aqueous layer was adjusted to 3 with activated Amberlyst 15 resin (30 gms). The resin was activated using concentrated Hydrochloric acid. The reaction mass was filtered and the clear filtrate was distilled under vacuum below 50°C to get crude 2S-Acetylamino-3-(2-oxo-cyclopentyl)-propionic acid. Butanone (150 ml) was added to crude 2S-acetylamino-3-(2'oxo-cyclopentyl)-propionic acid and suspension was heated to get clear solution. The solution was gradually cooled to get thick crystal slurry. The slurry was filtered and washed with butanone to get optically pure 2S-Acetylamino-3-(2-oxo-cyclopentyl)-propionic acid (Yield: 35 gms) (Purity: 98.8%; 97% ee). The separated MDC layer was distilled to get undesired D-isomer.

### Example 2

### Preparation of hydrochloric acid salt of (2S)-2,3,3a,4,5,6-hexahydro-cvclopenta[b]pyrrole-2-carboxylic acid (IV)

The product obtained in example 1 (15 gm) was dissolved in water (30 ml) and concentrated Hydrochloric acid (12 ml) was added. The mixture was heated to reflux and maintained for 3 hours at reflux temperature. MDC (3 X 45 ml) was added to the reaction solution and extracted thoroughly. The aqueous layer was distilled under vacuum below 45°C to get thick mass of hydrochloric acid salt of (2S)-2,3,3a,4,5,6-Hexahydro-cyclopenta[b]pyrrole-2-carboxylic acid (11 gm) (Purity: 99.1%; 97.3% ee)

### Example 3

### Prenaration of hydrochloric acid salt of (2S, 3aS, 6aS)-octahydrocyclopenta[b]pyrrole-2-carboxylic acid (C)

Hydrochloric acid salt of (2S)-2,3,3a,4,5,6-hexahydro-cyclopenta[b]pyrrole-2-carboxylic acid obtained in example 2 (10 gm) was added to acetic acid (60 ml) with constant stirring. Heat the reaction mixture at 40-45°C. 5% Pd/C (0.8 gm) was added to above reaction mixture and was hydrogenated under hydrogen pressure between 5-6 Kg/cm² at 55-60°C. After ensuring completion of the reaction the reaction mass was filtered to remove catalyst and concentrated. Acetone (20 ml) was added and the reaction mixture was stirred for 30 min at 30-35°C. The mixture was chilled to 0-5°C. The mass was filtered and dried. Hydrochloric acid salt of (2S, 3aS, 6aS)-octahydrocyclopenta[b]pyrrole-2-carboxylic acid was obtained as solid (6 gm) (Purity: 99.2%; 97.5% ee)

### Example 4

### Hydrochloric acid salt of Benzyl (2S,3aS,6aS)-octahydrocyclopenta[b]pyrrole-2-carboxylate (Ba)

Hydrochloric acid salt of (2S, 3aS; 6aS)-octahydrocyclopenta[b]pyrrole-2-carboxylic acid (5 gm) was dissolved in benzyl alcohol (50 ml) at 25-30°C with constant stirring. The solution was cooled to 0-5°C and thionyl chloride (5 ml) was added drop wise. After completion of thionyl chloride addition the reaction mixture was heated to 25-30°C and stirred continuously. After completion of the reaction, the mixture was cooled to 10-15°C. Di-isopropyl ether (110 ml) was added and the mixture for was maintained at 10-15°C for 2 hours. Hydrochloric acid salt of benzyl (2S,3a*S*,6aS)-octahydrocyclopenta[*b*]pyrrole-2-carboxylate (5 gm) obtained was centrifuged, dried and purified further. (Purity 99.2%; 98.5% ee)

### Example 5

### Recovery of methyl-2-(acetylamino)-3-(2-oxocyclopentyl)-propionate (II)

Methanol (150 ml) was added to undesired isomer (50 gm) obtained by distillation of organic layer of example 1. Sodium methoxide (1.2 gm) was added to the mass and stirred for 10 minutes. The reaction mixture was gradually heated to reflux and heating was continued for 1 hour. Methanol was distilled below 50°C and degassed under vacuum. Separately prepared solution of 10% sodium bi-carbonate was added to the reaction mass and stirred for 30 minutes. MDC (25 ml) was added to the aqueous layer and stirred for 30 minutes. The organic layer was separated and MDC was distilled below 35°C to isolate 2-acetylamino-3-(2-oxo-cyclopentyl)-propionic acid methyl ester (45 gm). The distereoisomeric mixture was recycled back for preparation of desired (+) isomer of (2S)-acetylamino-3-(2-oxo-cyclopentyl)-propionic acid. (Purity 98%)

### Example 6

### Preparation of (2S)-Acetylamino-3-(2-oxo-cyclopentyl)-propionic acid (I)

Methyl 2-(acetylamino)-3-(2-oxocyclopentyl)-propionate (100 gm) was dissolved in water (240 ml) with constant stirring to get a clear solution. pH of the solution was adjusted to 6.8 by separately prepared solution of 10% sodium carbonate. *Protex 6L* (5 gm) was added to the solution and with constant stirring, pH of the reaction mass was adjusted to 6.6 at 24 - 26°C by sodium carbonate solution. Reaction mass was maintained for 4-5 hours. pH of the solution was adjusted to 6 by separately prepared 10% Acetic acid solution. Reaction mass was extracted with MDC (3 X 280 ml). After separation of organic and aqueous layer, pH of the aqueous layer was adjusted to 3 with freshly prepared solution of 15% HCl. The reaction mixture was filtered and the filtrate containing (2S)-Acetylamino-3-(2-oxo-cyclopentyl)-propionic acid was taken for further step. The separated MDC layer was distilled to get undesired D-isomer (III).

### Example 7

### Preparation of Paratoluene sulfonic acid salt of (2S)-2,3,3a,4,5,6-hexahydrocyclopenta[b]pyrrole-2-carboxylic acid (IV)

To the filtrate obtained in example 6, para-toluene sulfonic acid (126 gm) was added. The reaction mixture was stirred for 30 min at 25-30°C. Gradually the temperature was raised to reflux. After completion of the reaction, the reaction mixture was extracted with MDC (50 ml X 3). Both the layers were separated and aqueous layer was distilled under vacuum at 55°C to get thick mass containing Paratoluene sulfonic acid salt of (2S)-2,3,3a,4,5,6-hexahydro-cyclopenta[b]pyrrole-2-carboxylic acid.

### Example 8

### Preparation of Para-toluene sulfonic acid salt of (2S, 3aS, 6aS)-octahydrocyclopenta[b]pyrrole-2-carboxylic acid (C)

Aqueous layer containing Para-toluene sulfonic acid salt of (2S)-2,3,3a,4,5,6-hexahydro-cyclopenta[b]pyrrole-2-carboxylic acid was added to hydrogenator. Aqueous slurry of palladium catalyst was added to the solution and heated to 55-60°C. Hydrogen pressure of 5.5-6 Kg/cm² was applied and the reaction mixture was hydrogenated completely. The mass was filtered and the clear filtrate containing Para-toluene sulfonic acid salt of (2S, 3aS, 6aS)-octahydrocyclopenta[b]pyrrole-2-carboxylic acid was collected.

### Example 9

**Hydrochloric acid salt of Benzyl (2S,3a*S*,6aS)-octahydrocyclopenta[*b*]pyrrole-2-carboxylate (Ba)** Clear filtrate containing Para-toluene sulfonic acid salt of 2,3,3a,4,5,6-octahydrocyclopenta[b]pyrrole -2-carboxylic acid was distilled under vacuum below 50°C. Benzyl alcohol (500 ml) was added to the solution at 40-55°C. The reaction mass was heated and water was distilled out under vacuum. During reaction MDC (500 ml) was charged at 15-20°C and the reaction mass was stirred and settled for 1 hour. Distilled water (300 ml) was charged at 15-20°C. To separated MDC layer brine solution (200 ml) was added and the reaction mass was chilled at 0-5°C. pH of the reaction mass was adjusted to 9 - 9.5 at 0-5°C. The reaction mass was stirred for 15 minutes. Separated MDC layer was distilled under vacuum below 30°C. The reaction mass was degassed under vacuum below 30°C. To the oil thus obtained acetone (300 ml) was added. The mixture was charcoalised and stirred for 30 minutes. The mass was filtered and clear filtrate was chilled to 0-5°C and pH was adjusted to -2.9 to -2.7 at 0-5°C by dry HCl gas. Centrifuge the mass and hydrochloric acid salt of Benzyl (2S,3a*S*,6aS)-octahydrocyclopenta [*b*]pyrrole-2-carboxylate (50 gm) was isolated. (Purity: 99.4%; 97.5% ee)

### Example 10

### Purification of Hydrochloric acid salt of Benzyl (2S,3aS,6aS)-octahydrocyclopenta[b]pyrrole-2-carboxylate

Hydrochloric acid addition salt of (2S, 3aS, 6aS)-octahydrocyclopenta[b]pyrrole-2-carboxylic acid benzyl ester (50 gm) obtained in the above example 4 was added to Di-isopriopyl ether (600 ml) at 25-30°C and the solution was stirred for 10 min. The solution is chilled to 5-10°C and pH of the solution was adjusted to 9-10 with separately prepared 10% sodium carbonate solution. The mixture was stirred for 30 minutes and allowed to settle. The layers were separated and Di-isopriopyl ether (2 X 100 ml) in aqueous layer and stirred for 15 minutes. Organic layers were collected and chilled at 5-10°C. DIPE-HCl was added to the reaction mass till the pH is 2-3 at 5-10°C. The mass was stirred continuously and centrifuged to get pure Hydrochloric acid addition salt of (2S, 3aS, 6aS)-octahydrocyclopenta[b] pyrrole-2-carboxylic acid benzyl ester (40 gm) (Purity: 99.3%; 98.2% ee)

### Example 11

### Preparation of [2S, 3aS, 6aS]-1-[(2S)-2-[[(1S)-1-(Ethoxycarbonyl)-3-phenylpropyl]amino]-1-oxopropyl]octahydrocyclopenta[b]pyrrole-2-carboxylic acid [Ramipril]

Toluene (400 ml) was added to 1-hydroxybenzotriazole (26.6 gm) and stirred for 15 minutes. N-(1-S-carbethoxy-3-phenyl propyl)-S-alanine (44 gm) and optically pure hydrochloric acid addition salt of (2S, 3aS, 6aS)-octahydrocyclopenta[b]pyrrole-2-carboxylic acid benzyl ester (40 gms) obtained from example 9 was added to the reaction mixture at 0-5°C. N-methyl morpholine (15.8 gms) was added and the reaction mixture was stirred for 5 minutes. Dicyclohexyl carbodiimide (32.8 gms) was added and the mixture was stirred for 60 minutes at 0-5°C. After completion of the reaction, reaction mass was filtered. Toluene layer was concentrated and acetone (95 ml) was added and mixture was stirred at 0-5°C. The solution was filtered and distilled under vacuum below 40°C. Thick yellow oil (72 gm) of benzyl ester of Ramipril was obtained. Ethanol (640 ml) was added to the ester. 5% Pd/C was added to the reaction mixture and hydrogenation was carried out at 20-22°C at 45-50 psi hydrogen pressure. The mass was filtered and the filtrate was distilled and degassed under vacuum below 25°C. [2*S*, 3a*S*, 6a*S*]-1-[(2*S*)-2-[[(1*S*)-1-(Ethoxycarbonyl)-3-phenylpropyl]amino]-1-oxopropyl] octahydrocyclopenta[*b*] pyrrole-2-carboxylic acid obtained was isolated by DIPE (480 ml) at 0-15°C. The solid was dried at 30°C under vacuum. (wt. on dry basis: 50.2 gms) (Purity: 99.7%; 98.1% ee)

## Claims

1. A process for Enantio-specific synthesis of optically pure (2S)-acetylamino-3-(2-oxocyclopentyl)-propionic acid of formula (I) comprising: 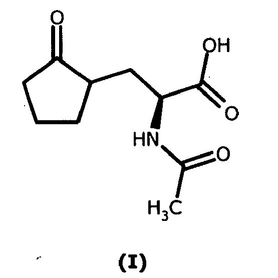 hydrolyzing stereoisomeric mixture of (1-4C alkyl)-2-(acetylamino)-3-(2-oxocyclopentyl) propanoate of formula (II), in which R is (1-4C) alkyl, 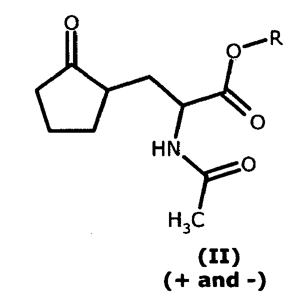 with an Alkaline serine endopeptidase enzyme, to form a mixture of (+) isomer (2S)-acetylamino-3-(2-oxo-cyclopentyl)-propionic acid (I) and (-) isomer (1-4C alkyl) (2R)-2-(acetylamino)-3-(2-oxocyclopentyl) propanoate (III)

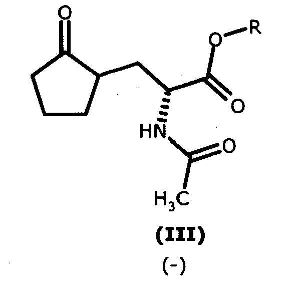

2. The process as claimed in claim 1, wherein the Alkaline serine endopeptidase enzyme is immobilized.

3. The process as claimed in claim 1, wherein the reaction is carried out at a temperature between 20-30°C.

4. The process as claimed in claim 3, wherein the reaction is carried out at a temperature between 24-26°C.

5. The process as claimed in claim 1, wherein the pH of the reaction is between 6-7.

6. The process as claimed in claim 5, wherein the pH of the reaction is between 6.2 - 6.8.

7. The process as claimed in claim 5, wherein the pH is adjusted with an alkali, selected from sodium carbonate and sodium hydroxide.

8. The process as claimed in claim 1, wherein the (-) isomer (III) is separated by extraction with an organic solvent selected from a group of dichloromethane, ethylene dichloride, ethyl acetate and tri-chloro methane to form an optically pure isomer (I).

9. The process as claimed in claim 1, which further comprises, recovery and racemization of the isomer (III) to stereoisomeric mixture II, by:
i. concentrating the organic layer;
ii. treating the residue with suitable alcohol and refluxing the mixture;
iii. treating the reaction mass with suitable alkali alkoxide;
iv. heating the mixture to reflux and gradually cooling the mixture; and
v. stirring the reaction mass and isolation of the racemic compound (II) (+ and -) by distillation.

10. The process as claimed in claim 9, wherein the alcohol used in step (ii) is selected from a group of methanol, ethanol, isopropanol and butanol.

11. The process as claimed in claim 10, wherein the alcohol is methanol.

12. The process as claimed in claim 9, wherein the alkali alkoxide used in step (iii) is selected from a group of sodium methoxide, sodium ethoxide, sodium propoxide, sodium butoxide, potassium methoxide, potassium ethoxide and potassium butoxide.

13. The process as claimed in claim 12, wherein the alkali alkoxide used in step (iii) is preferably sodium methoxide.

14. The process as claimed in claim 9, the recovered and recemized isomer (II) (+ and -) obtained from step (b) is recycled in step a for enantio-specific synthesis of optically pure intermediate (I).

15. The process as claimed in any of preceding claims, wherein the recovery of Immozyme Alkaline serine endopeptidase comprises:
i. Immozyme being washed thoroughly with cold acetone and dried under air or nitrogen stream, and stored at around 4°C; or
ii. Immozyme being washed with 50% aqueous 1,2-propanediol and stored in this form at -18°C and before re-use the Immozyme is warmed to 0-5°C and washed thoroughly with water prior to use to remove 1,2-propanediol traces.

16. The process as claimed in any of the preceding claims, which further comprises, preparation of cis endo 2-azabicyclo[3.3.0]-octane-3S-carboxylic acid or its ester (Ba), by:
i) hydrolytic cyclization of optically pure intermediate (I) obtained in step a) in any of the above claims, by refluxing (I) in presence of suitable cyclizing agent; 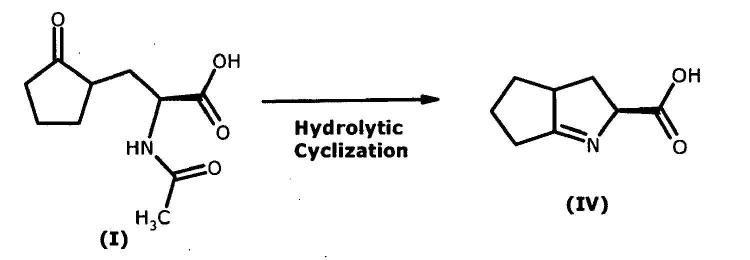
i. reduction to get acid (C); and optionally
ii. esterification to get optically pure (Ba)

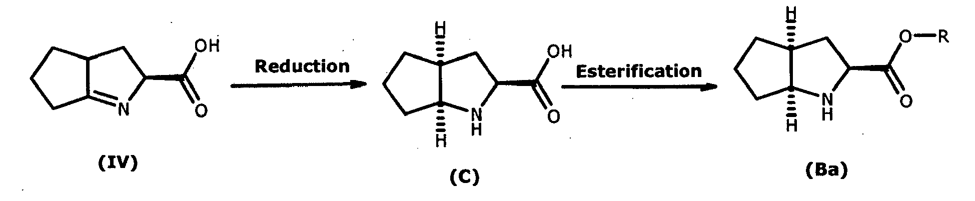

17. A process for preparation of cis endo 2-azabicyclo[3.3.0]-octane-3S-carboxylic acid or its ester (Ba) comprises,
(A) enantio-specific synthesis of optically pure (2S)-acetylamino-3-(2-oxocyclopentyl)-propionic acid of the formula (I), 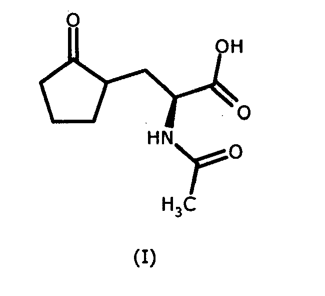 hydrolyzing stereoisomeric mixture of (1-4C alkyl)-2-(acetylamino)-3-(2-oxocyclopentyl) propanoate of the formula (II), 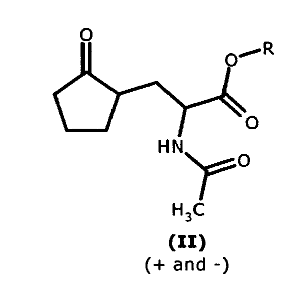 in which R is (1-4C) alkyl, with Protex 6L, to form mixture of (+) isomer (2S)-acetylamino-3-(2-oxo-cyclopentyl)-propionic acid (I) and (-) isomer (1-4C alkyl) (2R)-2-(acetylamino)-3-(2-oxocyclopentyl) propanoate (III) 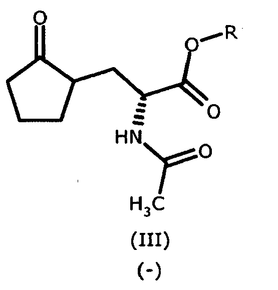
(B) separation of undesired (-) isomer (III) from optically pure isomer (I) by extracting with an organic solvent selected from dichloromethane, ethyl acetate and trichloro methane,
(C) recovery and racemization of the undesired isomer (III) to stereoisomeric mixture II, by
i. concentrating the organic layer;
ii. treating the residue with suitable alcohol and refluxing the mixture;
iii. treating the reaction mass with suitable alkali alkoxide;
iv. heating the mixture to reflux and gradually cooling the mixture; and
v. stirring the reaction mass and isolation of the racemic compound (II) (+ and -) by distillation.
vi. recycling racemic compound (II) (+ and -) obtained in step (v) to step (A)
(D) hydrolytic cyclization of optically pure intermediate (I) obtained in step (A) by refluxing (I) in presence of suitable cyclizing agent; 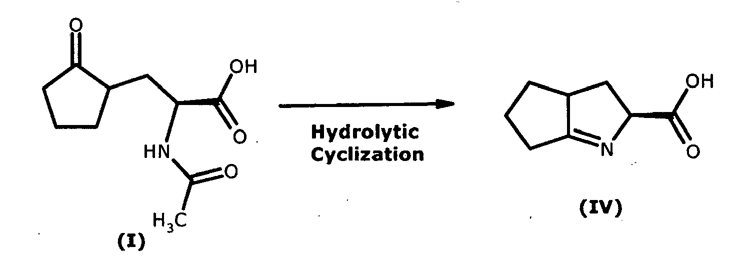
i. reduction to get acid (C); and optionally
ii. esterification to get optically pure (Ba)

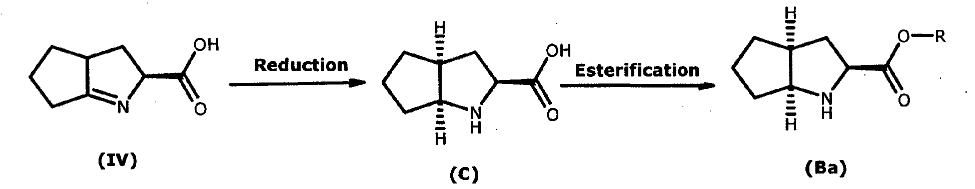

18. The process as claimed in claim 16 or 17, wherein cyclization step is carried out by refluxing (I) in presence of suitable cyclizing agent e.g. concentrated hydrochloric acid, sulfuric acid, para-toluene sulfonic acid and methane sulfonic acid.

19. The process as claimed in claims 16-18, further comprises preparation of Ramipril, by,
i) condensing ester of formula (Ba) formed in any of the claims 11-13, with (2S)-2-([(2S)-1-ethoxy-1-oxo-4-phenylbutan-2-yl]amino) propanoic acid to form Ramipril ester; and deprotection preferably by reduction with suitable hydrogenating agent 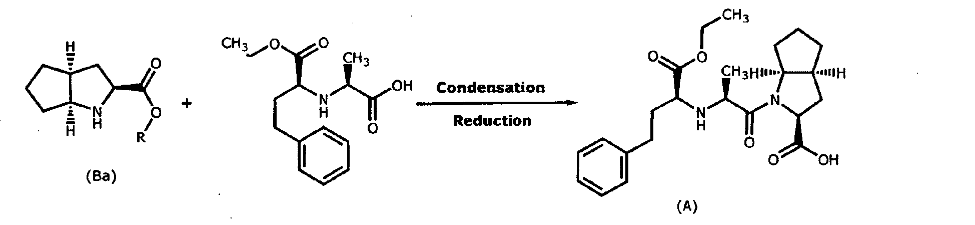 or;
ii) reacting intermediate octahydro-cyclopenta[b]pyrrole-2-carboxylic acid (IIa), with compound of formula (D), to give Ramipril (A)

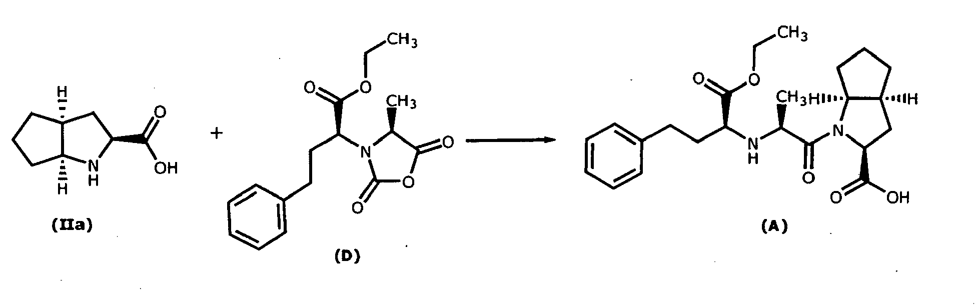

20. A process for preparation of Ramipril comprising:
(A) enantio-specific synthesis of optically pure (2S)-acetylamino-3-(2-oxocyclopentyl)-propionic acid of the formula (I) comprising: 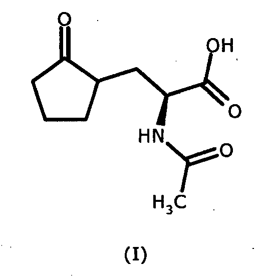 hydrolyzing stereoisomeric mixture of (1-4C alkyl)-2-(acetylamino)-3-(2-oxocyclopentyl)propanoate of the formula (II), in which R is (1-4C) alkyl 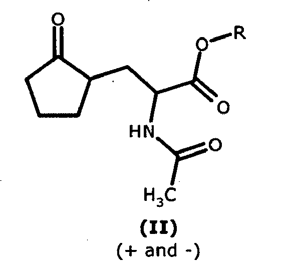 with Alkaline serine endopeptidase, to form mixture of (+) isomer (2S)-acetylamino-3-(2-oxo-cyclopentyl)-propionic acid of the formula (I) and (-) isomer (1-4C alkyl) (2R)-2-(acetylamino)-3-(2-oxocyclopentyl) propanoate of the formula (III) 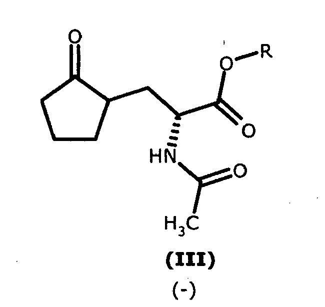
B) separation of undesired (-) isomer (III) from optically pure isomer (I) by extracting with an organic solvent selected from dichloromethane, ethyl acetate and trichloro methane.
(C) recovery and racemization of the undesired isomer (III) to stereoisomeric mixture II, by
i. concentrating the organic layer;
ii. treating the residue with suitable alcohol and refluxing the mixture;
iii. treating the reaction mass with suitable alkali alkoxide;
iv. heating the mixture to reflux and gradually cooling the mixture; and
v. stirring the reaction mass and isolation of the racemic compound (II) (+ and -) by distillation
vi. recycling racemic compound (II) (+ and -) obtained in step (v) to step (A)
(D) hydrolytic cyclization of optically pure intermediate (I) obtained in step (A) by refluxing (I) in presence of suitable cyclizing agent; 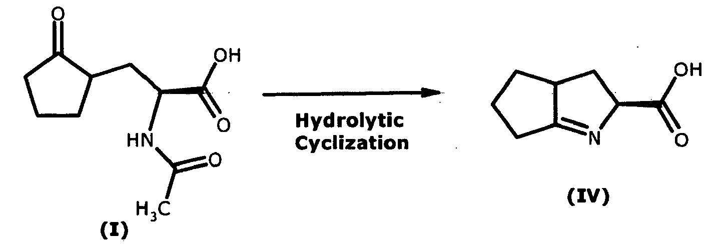
i. reduction to get acid (C); and optionally
ii. esterification to get optically pure (Ba) 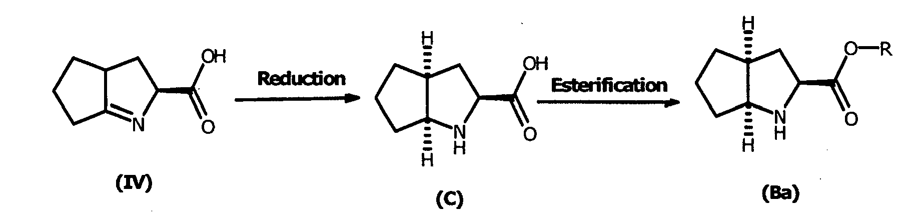
(E) condensing ester of formula (Ba) formed in step (C), with (2S)-2-([(2S)-1-ethoxy-1-oxo-4-phenylbutan-2-yl]amino)propanoic acid to form Ramipril ester; and deprotection preferably by reduction with suitable hydrogenating agent; 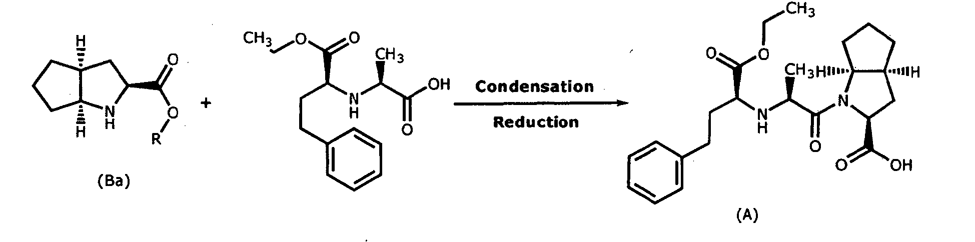 or;
reacting intermediate octahydro-cyclopenta [b] pyrrole-2-carboxylic acid (IIa), with compound of formula (D), to give Ramipril (A):

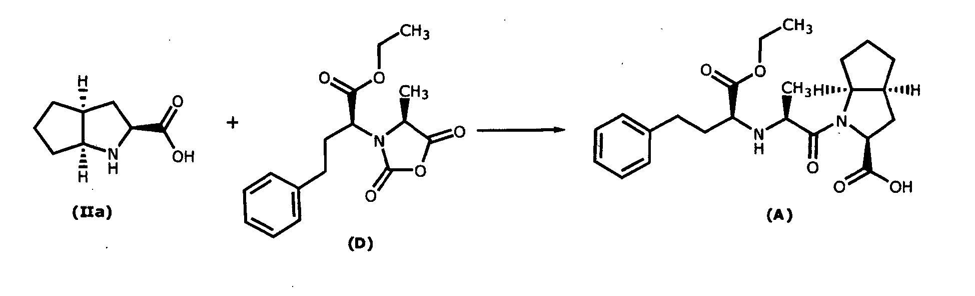

## Patentansprüche

1. Verfahren zur enantiospezifischen Synthese von optisch reiner (2S)-acetylamino-3-(2-oxocyclopentyl)-propionsäure der Formel (I), umfassend: 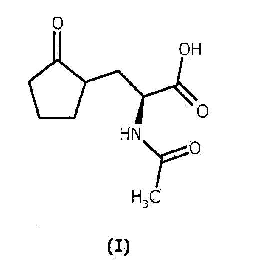 Hydrolysieren einer stereoisomeren Mischung von (1-4C-Alkyl)-2-(acetylamino)-3-(2-oxocyclopentyl)-propanoat der Formel (II), in welcher R (1-4C)-Alkyl ist, 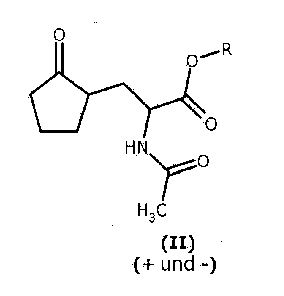 mit einem alkalischen Serin-Endopeptidase-Enzym, um eine Mischung aus (+)-Isomer der (2S)-acetylamino-3-(2-oxocyclopentyl)-propionsäure (I) und (-)-Isomer des (1-4C-Alkyl)-(2R)-2-(acetylamino)-3-(2-oxocyclopentyl)-propanoats (III) zu bilden.

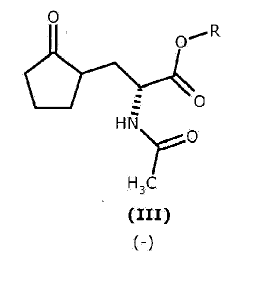

2. Verfahren nach Anspruch 1, **dadurch gekennzeichnet, dass** das alkalische Serin-Endopeptidase-Enzym immobilisiert ist.

3. Verfahren nach Anspruch 1, **dadurch gekennzeichnet, dass** die Reaktion bei einer Temperatur zwischen 20 - 30°C durchgeführt wird.

4. Verfahren nach Anspruch 3, **dadurch gekennzeichnet, dass** die Reaktion bei einer Temperatur zwischen 24 - 26°C durchgeführt wird.

5. Verfahren nach Anspruch 1, **dadurch gekennzeichnet, dass** der pH der Reaktion zwischen 6 - 7 ist.

6. Verfahren nach Anspruch 5, **dadurch gekennzeichnet, dass** der pH der Reaktion zwischen 6.2 - 6.8 ist.

7. Verfahren nach Anspruch 5, **dadurch gekennzeichnet, dass** der pH mit einem alkalischen Reagenz, das ausgewählt ist aus Natriumcarbonat und Natriumhydroxid, eingestellt wird.

8. Verfahren nach Anspruch 1, **dadurch gekennzeichnet, dass** das (-)-Isomer (III) durch Extraktion mit einem organischem Solvens, das ausgewählt ist aus einer Gruppe aus Dichlormethan, Ethylendichlorid, Ethylacetat und Trichlormethan, separiert wird, um ein optisch reines Isomer (I) zu bilden.

9. Verfahren nach Anspruch 1, weiterhin umfassend Rückgewinnung und Razemisierung des Isomers (III) zur stereisomeren Mischung II durch:
i. Aufkonzentrieren der organischen Phase;
ii. Behandeln des Rückstandes mit geeignetem Alkohol und behandeln der Mischung unter Rückfluss;
iii. Behandeln der Reaktionsmasse mit geeignetem Alkali-Alkoxid;
iv. Erwärmen der Mischung zum Rückfluss und allmäliches Abkühlen der Mischung; und
v. Rühren der Reaktionsmasse und Isolieren der racemischen Verbindung (II) (+ und -) durch Destillation.

10. Verfahren nach Anspruch 9, **dadurch gekennzeichnet, dass** der Alkohol, der in Schritt (ii) verwendet wird aus einer Gruppe aus Methanol, Ethanol, Isopropanol und Butanol ausgewählt ist.

11. Verfahren nach Anspruch 10, **dadurch gekennzeichnet, dass** der Alkohol Methanol ist.

12. Verfahren nach Anspruch 9, **dadurch gekennzeichnet, dass** das Alkali-Alkoxid, das in Schritt (iii) verwendet wird aus einer Gruppe aus Natriummethanolat, Natriumethanolat, Natriumpropanolat, Natriumbutanolat, Kaliummethanolat, Kaliumethanolat und Kaliumbutanolat ausgewählt ist.

13. Verfahren nach Anspruch 12, **dadurch gekennzeichnet, dass** das Alkali-Alkoxid bevorzugt Natriummethanolat ist.

14. Verfahren nach Anspruch 9, **dadurch gekennzeichnet, dass** das rückgewonnene und razemisierte Isomer (II) (+ und -) welches aus Schritt (b) erhalten wurde in Schritt a zur enantiospezifischen Synthese von optisch reinem Intermediat (I) recycelt wird.

15. Verfahren nach einem der vorangegangenen Ansprüche, **dadurch gekennzeichnet, dass** die Rückgewinnung von Immozym alkalische Serin-Endopeptidase umfasst:
i. dass Immozym gründlich mit kaltem Aceton gewaschen und unter einem Luft- oder Stickstoffstrom getrocknet und bei ungefähr 4°C gelagert wird; oder
ii. dass Immozym mit 50%igem, wässrigem 1,2-Popandiol gewaschen und in dieser Form bei -18°C gelagert und vor Wiederverwendung das Immozym auf 0 - 5°C erwärmt und vor Verwendung gründlich mit Wasser gewaschen wird, um 1,2-Popandiolreste zu entfernen.

16. Verfahren nach einem der vorangegangenen Ansprüche, weiterhin umfassend die Herstellung von cis-endo-2-Azabicyclo[3.3.0]-oktan-3S-carboxylsäure oder dessen Ester (Ba) durch:
i. hydrolytische Cyclisierung von optisch reinem Intermediat (I), welches in Schritt (A) gemäß einem der vorangegangenen Ansprüche erhalten wurde, durch Behandlung unter Rückfluss von (I) in Anwesenheit eines geeigneten Cyclisierungsmittels; 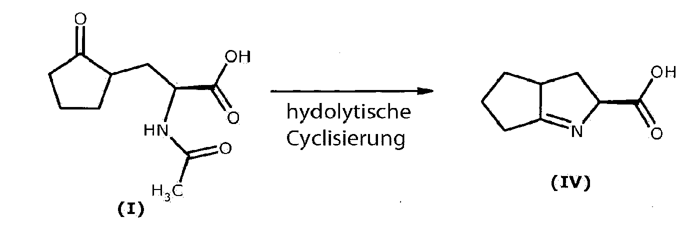
i. Reduktion um Säure (C) zu erhalten; und optional
ii. Veresterung um optisch reines (Ba) zu erhalten.

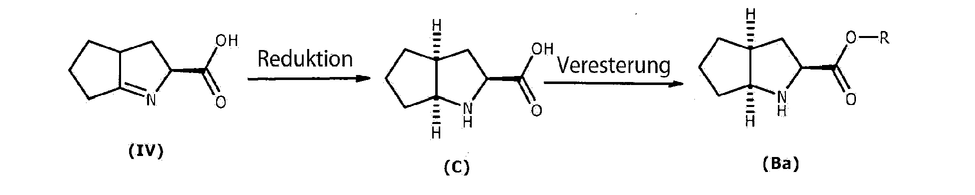

17. Verfahren zur Herstellung von cis-endo-2-Azabicyclo[3.3.0]-oktan-3S-carboxylsäure oder dessen Ester (Ba) umfassend
(A) enantiospezifische Synthese von optisch reinem (2S)-acetylamino-3-(2-oxocyclopentyl)-propionsäure der Formel (I), 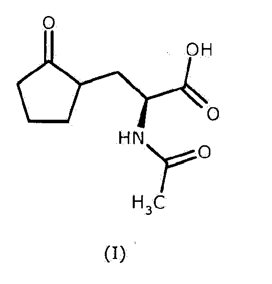 Hydrolysieren einer stereoisomeren Mischung von (1-4C-Alkyl)-2-(acetylamino)-3-(2-oxocyclopentyl)-propanoat der Formel (II), 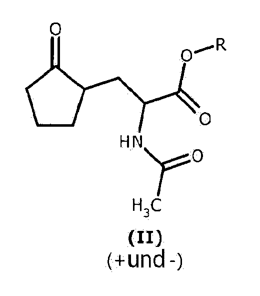 in welcher R (1-4C)-Alkyl ist, mit Protex 6L, um eine Mischung aus (+)-Isomer der (2S)-acetylamino-3-(2-oxocyclopentyl)-propionsäure (I) und (-)-Isomer des (1-4C-Alkyl)-(2R)-2-(acetylamino)-3-(2-oxocyclopentyl)-propanoats (III) zu bilden, 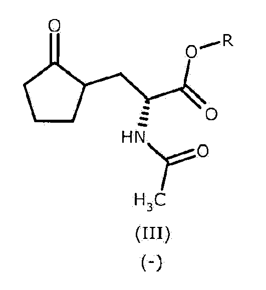
(B) Separierung von unerwünschtem (-)-Isomer (III) von optisch reinem Isomer (I) durch Extraktion mit einem organischen Solvens, welches ausgewählt ist aus Dichlormethan, Ethylacetat und Trichlormethan,
(C) Rückgewinnung und Razemisierung des unerwünschten Isomers (III) zur stereisomeren Mischung II durch:
i. Aufkonzentrieren der organischen Phase;
ii. Behandeln des Rückstandes mit geeignetem Alkohol und behandeln der Mischung unter Rückfluss;
iii. Behandeln der Reaktionsmasse mit geeignetem Alkali-Alkoxid;
iv. Erwärmen der Mischung zum Rückfluss und allmähliches Abkühlen der Mischung; und
v. Rühren der Reaktionsmasse und Isolieren der racemischen Verbindung (II) (+ und -) durch Destillation;
vi. Recycling der racemischen Verbindung (II) (+ und -), welches in Schritt (v) erhalten wurde zu Schritt (A)
(D) hydrolytische Cyclisierung von optisch reinem Intermediat (I), welches in Schritt (A) erhalten wurde durch Behandlung unter Rückfluss von (I) in Anwesenheit eines geeigneten Cyclisierungsmittels; 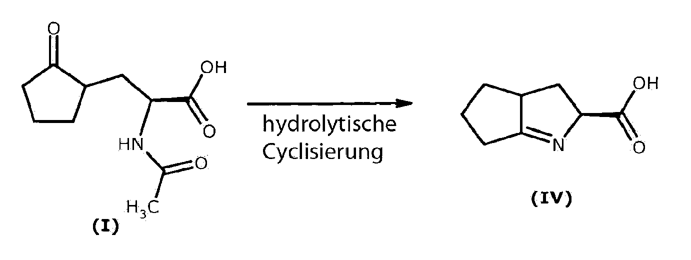
i. Reduktion um Säure (C) zu erhalten; und optional
ii. Veresterung um optisch reines (Ba) zu erhalten.

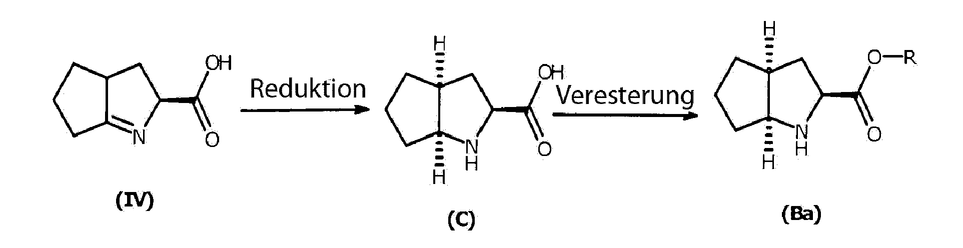

18. Verfahren nach Anspruch 16 oder 17, **dadurch gekennzeichnet, dass** der Cyclisierungsschritt durch Behandlung unter Rückfluss von (I) in Gegenwart eines geeigneten Cyclisierungsreagenzes wie z.B. konzentrierte Salzsäure, Schwefelsäure, para-Toluolsulfonsäure und Methansulfonsäure durchgeführt wird.

19. Verfahren nach einem der Ansprüche 16 - 18, weiterhin umfassend die Herstellung von Ramipril durch
i) Kondensieren des Esters nach Formel (Ba), gebildet nach einem der Ansprüche 11- 13, mit (2S)-2-([(2S)-1-ethoxy-1-oxo-4-phenylbutan-2-yl]amino)-propionsäure um Ramiprilester zu bilden; und Entschützung, bevorzugt durch Reduktion mit einem geeigneten Hydrierungsmittel 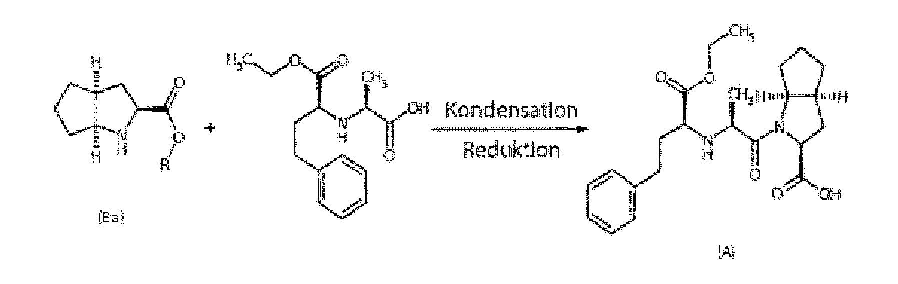 oder;
ii) Umsetzen des Intermediates Octahydro-cyclopenta-[b]pyrrol-2-carboxylsäure (IIa) mit der Verbindung der Formel (D), um Ramipril (A) zu bilden.

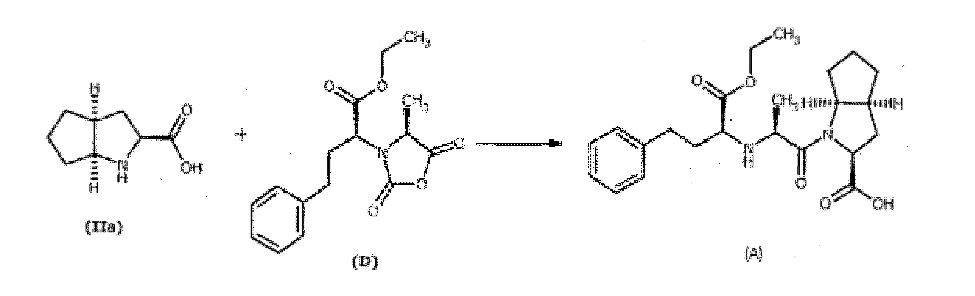

20. Verfahren zur Herstellung von Ramipril umfassend:
(A) enantiospezifische Synthese von optisch reiner (2S)-acetylamino-3-(2-oxocyclopentyl)-propionsäure der Formel (I), 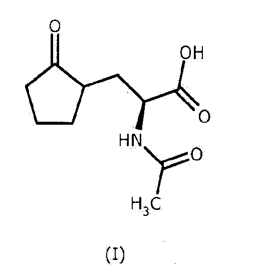 Hydrolysieren einer stereoisomeren Mischung von (1-4C-Alkyl)-2-(acetylamino)-3-(2-oxocyclopentyl)-propanoat der Formel (II), in welcher R (1-4C)-Alkyl ist, 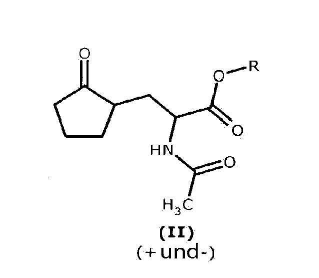 mit einem alkalischer Serin-Endopeptidase, um eine Mischung aus (+)-Isomer der (2S)-acetylamino-3-(2-oxo-cyclopentyl)-propionsäure der Formel (I) und (-)-Isomer des (1-4C-Alkyl)-(2R)-2-(acetylamino)-3-(2-oxocyclopentyl)-propanoats der Formel (III) zu bilden 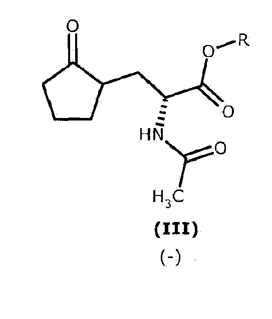
(B) Separierung von unerwünschtem (-)-Isomer (III) von optisch reinem Isomer (I) durch Extraktion mit einem organischen Solvens, welches ausgewählt ist aus Dichlormethan, Ethylacetat und Trichlormethan,
(C) Rückgewinnung und Razemisierung des unerwünschten Isomers (III) zur stereisomeren Mischung (II) durch:
i. Aufkonzentrieren der organischen Phase;
ii. Behandeln des Rückstandes mit geeignetem Alkohol und behandeln der Mischung unter Rückfluss;
iii. Behandeln der Reaktionsmasse mit geeignetem Alkali-Alkoxid;
iv. Erwärmen der Mischung zum Rückfluss und allmähliches Abkühlen der Mischung; und
v. Rühren der Reaktionsmasse und Isolieren der racemischen Verbindung (II) (+ und -) durch Destillation;
vi. Recycling der racemischen Verbindung (II) (+ und -), welches in Schritt (v) erhalten wurde zu Schritt (A);
(D) hydrolytische Cyclisierung von optisch reinem Intermediat (I), welches in Schritt (A) erhalten wurde, durch Behandlung unter Rückfluss von (I) in Anwesenheit eines geeigneten Cyclisierungsmittels; 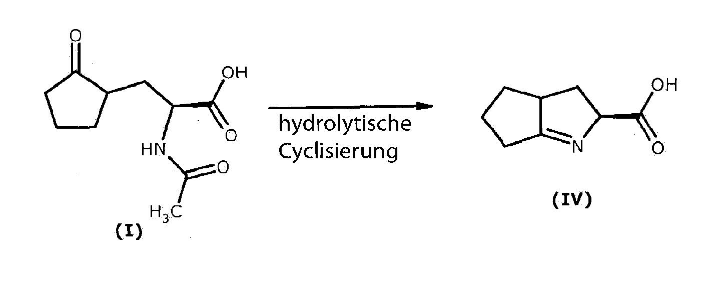
i. Reduktion um Säure (C) zu erhalten; und optional
ii. Veresterung um optisch reines (Ba) zu erhalten 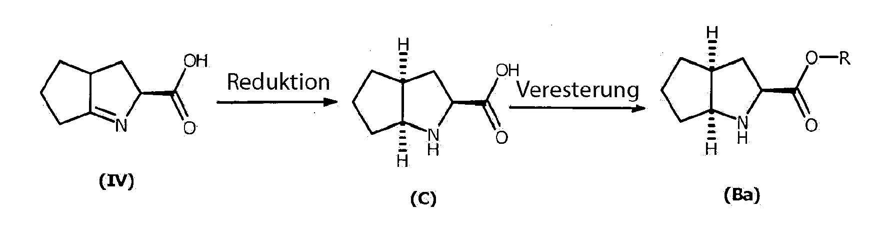
(E) Kondensieren des Esters nach Formel (Ba), gebildet in Schritt (C), mit (2S)-2-([(2S)-1-ethoxy-1-oxo-4-phenylbutan-2-yl]amino)-propionsäure um Ramiprilester zu bilden; und Entschützung, bevorzugt durch Reduktion mit einem geeigneten Hydrierungsmittel 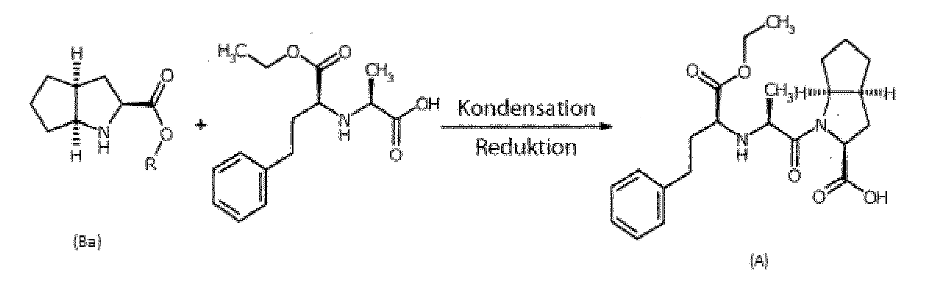 oder;
Umsetzen des Intermediates Octahydro-cyclopenta-[b]-pyrrol-2-carboxylsäure (IIa) mit der Verbindung der Formel (D), um Ramipril (A) zu bilden:

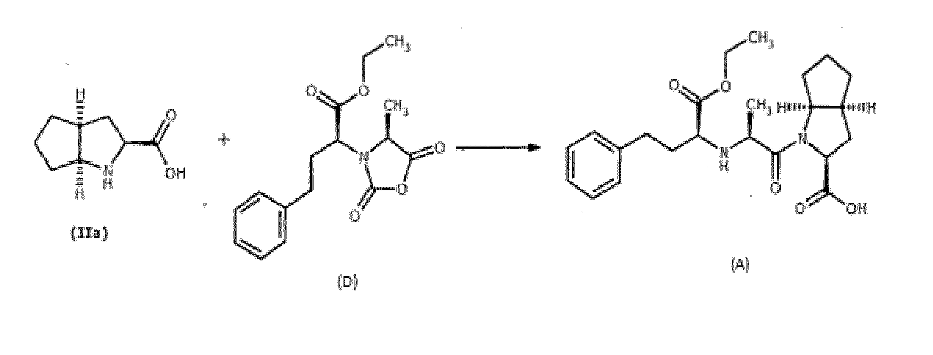

## Revendications

1. Procédé de synthèse énantiospécifique de l'acide (2S)-acétylamino-3-(2-oxocyclopentyl)-propionique optiquement pur, de formule (I), comprenant : 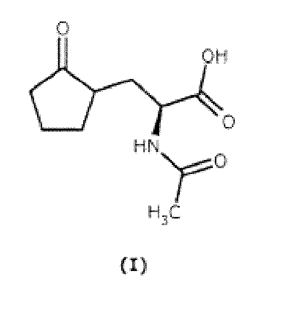 l'hydrolyse d'un mélange stéréoisomérique d'un propanoate d'(alkyle en C1-4)-2-(acétylamino)-3-(2-oxocyclopentyle) de formule (II), dans laquelle R est un alkyle (en C1-4), 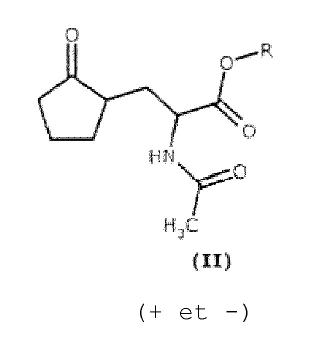 avec une enzyme sérine endopeptidase alcaline, pour former un mélange de l'isomère (+) de l'acide (2S)-acétylamino-3-(2-oxocyclopentyl)-propionique (I) et de l'isomère (-) du propanoate d'(alkyle en C1-4) (2R)-2-(acétylamino)-3-(2-oxocyclopentyle) (III)

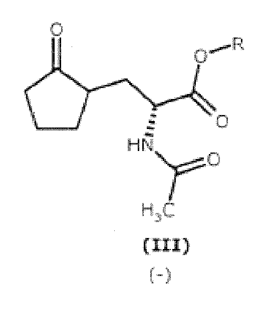

2. Procédé selon la revendication 1, dans lequel l'enzyme sérine endopeptidase alcaline est immobilisée.

3. Procédé selon la revendication 1, dans lequel la réaction est réalisée à une température entre 20 et 30 °C.

4. Procédé selon la revendication 3, dans lequel la réaction est réalisée à une température entre 24 et 26 °C.

5. Procédé selon la revendication 1, dans lequel le pH de la réaction est entre 6 et 7.

6. Procédé selon la revendication 5, dans lequel le pH de la réaction est entre 6,2 et 6,8.

7. Procédé selon la revendication 5, dans lequel le pH est ajusté avec un alcali choisi parmi le carbonate de sodium et l'hydroxyde de sodium.

8. Procédé selon la revendication 1, dans lequel l'isomère (-) (III) est séparé par extraction avec un solvant organique choisi dans le groupe constitué par le dichlorométhane, le dichlorure d'éthylène, l'acétate d'éthyle et le trichlorométhane pour former un isomère optiquement pur (I).

9. Procédé selon la revendication 1, comprenant en outre la récupération et la racémisation de l'isomère (III) en mélange stéréoisomérique II, par :
i. concentration de la couche organique ;
ii. traitement du résidu avec un alcool approprié et mise au reflux du mélange ;
iii. traitement de la masse réactionnelle avec un alcoxyde d'alcali approprié ;
iv. chauffage du mélange au reflux et refroidissement progressif du mélange ; et
v. agitation de la masse réactionnelle et isolement du composé racémique (II) (+ et -) par distillation.

10. Procédé selon la revendication 9, dans lequel l'alcool utilisé à l'étape (ii) est choisi dans un groupe constitué par le méthanol, l'éthanol, l'isopropanol et le butanol.

11. Procédé selon la revendication 10, dans lequel l'alcool est le méthanol.

12. Procédé selon la revendication 9, dans lequel l'alcoxyde d'alcali utilisé à l'étape (iii) est choisi dans un groupe constitué par le méthoxyde de sodium, l'éthoxyde de sodium, le propoxyde de sodium, le butoxyde de sodium, le méthoxyde de potassium, l'éthoxyde de potassium et le butoxyde de potassium.

13. Procédé selon la revendication 12, dans lequel l'alcoxyde d'alcali utilisé à l'étape (iii) est de préférence le méthoxyde de sodium.

14. Procédé selon la revendication 9, dans lequel l'isomère récupéré et racémisé (II) (+ et -) obtenu à l'étape (b) est recyclé dans une étape de synthèse énantiospécifique de l'intermédiaire optiquement pur (I).

15. Procédé selon l'une quelconque des revendications précédentes, dans lequel la récupération de l'immozyme sérine endopeptidase alcaline comprend :
i. le lavage abondant de l'immozyme avec de l'acétone froide et son séchage sous un courant d'air ou d'azote, et son stockage à environ 4 °C ; ou
ii. le lavage de l'immozyme avec du 1,2-propanediol aqueux à 50 % et son stockage sous cette forme à -18 °C et, avant réutilisation, le chauffage de l'immozyme à 0-5 °C et son lavage abondant avec de l'eau avant utilisation pour éliminer les traces de 1,2-propanediol.

16. Procédé selon l'une quelconque des revendications précédentes, comprenant en outre la préparation de l'acide cis endo 2-aza-bicyclo[3.3.0]-octane-3S-carboxylique ou de son ester (Ba), par :
i. cyclisation hydrolytique de l'intermédiaire optiquement pur (I) obtenu à l'étape (A) dans l'une quelconque des revendications précédentes, par mise au reflux de (I) en présence d'un agent de cyclisation approprié ; 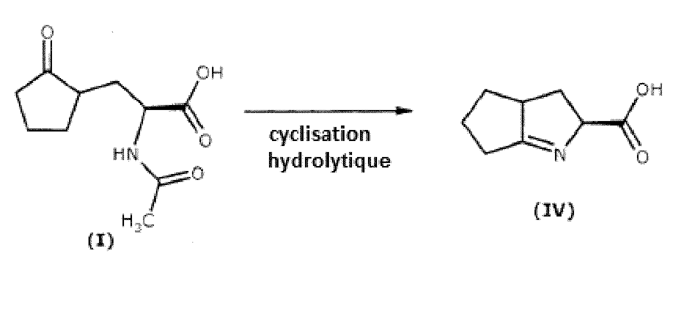
ii. réduction pour obtenir l'acide (C) ; et facultativement
iii. estérification pour obtenir (Ba) optiquement pur

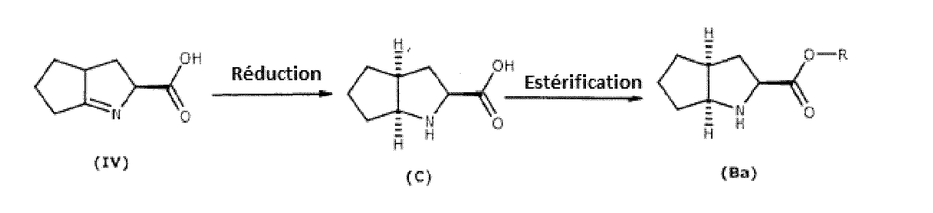

17. Procédé de préparation de l'acide cis endo 2-azabicyclo[3.3.0]-octane-3S-carboxylique ou de son ester (Ba), comprenant
(A) la synthèse énantiospécifique de l'acide (2S)-acétylamino-3-(2-oxocyclopentyl)-propionique optiquement pur de formule (I), 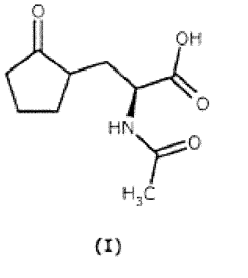 l'hydrolyse d'un mélange stéréoisomérique d'un propanoate d'(alkyle en C1-4)-2-(acétylamino)-3-(2-oxocyclopentyle) de formule (II), 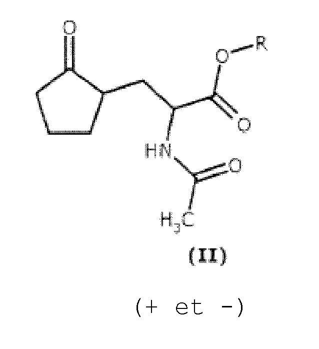 dans laquelle R est un alkyle (en C1-4), avec du Protex 6L, pour former un mélange de l'isomère (+) de l'acide (2S)-acétylamino-3-(2-oxocyclopentyl)-propionique (I) et de l'isomère (-) du propanoate d'(alkyle en C1-4) (2R)-2-(acétylamino)-3-(2-oxocyclopentyle) (III) 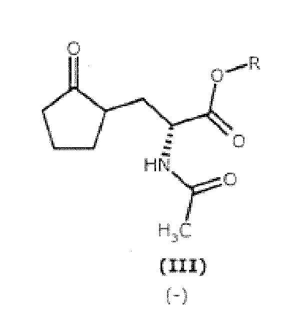
(B) séparation de l'isomère (-) (III) non souhaité de l'isomère optiquement pur (I) par extraction avec un solvant organique choisi parmi le dichlorométhane, l'acétate d'éthyle et le trichlorométhane,
(C) récupération et racémisation de l'isomère (III) non souhaité en le mélange stéréoisomérique II, par
i. concentration de la couche organique ;
ii. traitement du résidu avec un alcool convenable et mise au reflux du mélange ;
iii. traitement de la masse réactionnelle avec un alcoxyde d'alcali approprié ;
iv. chauffage du mélange au reflux et refroidissement progressif du mélange ; et
v. agitation de la masse réactionnelle et isolement du composé racémique (II) (+ et -) par distillation ;
vi. recyclage du composé racémique (II) (+ et -) obtenu à l'étape (v) vers l'étape (A)
(D) cyclisation hydrolytique de l'intermédiaire (I) optiquement pur obtenu à l'étape (A) par mise au reflux de (I) en présence d'un agent de cyclisation approprié ; 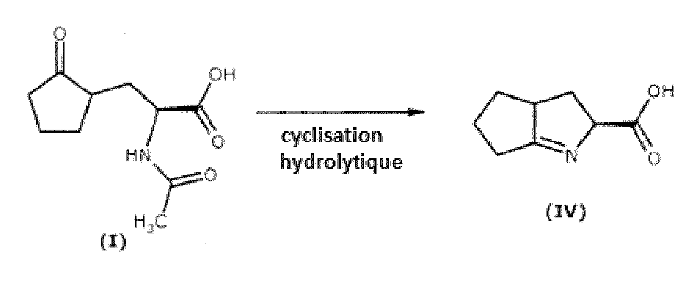
i. réduction pour obtenir l'acide (C) ; et facultativement
ii. estérification pour obtenir (Ba) optiquement pur

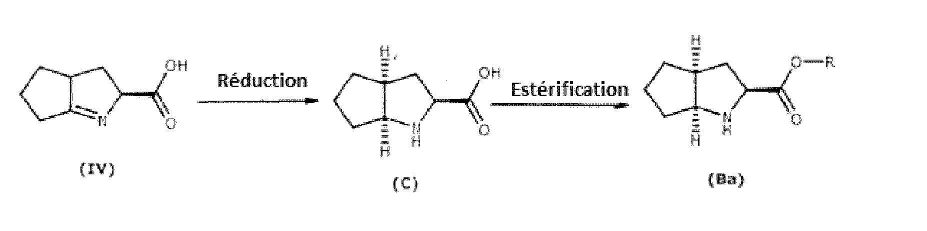

18. Procédé selon la revendication 16 ou 17, dans lequel l'étape de cyclisation est réalisée par mise au reflux de (I) en présence d'un agent de cyclisation approprié, par exemple de l'acide chlorhydrique, de l'acide sulfurique, de l'acide para-toluène sulfonique et acide méthane sulfonique concentré.

19. Procédé selon les revendications 16 à 18, comprenant en outre la préparation de ramipril, par :
i) condensation de l'ester de formule (Ba) formé dans l'une quelconque des revendications 11 à 13, avec de l'acide (2S)-2-([(2S)-1-éthoxy-1-oxo-4-phénylbutan-2-yl]amino)propanoïque pour former un ester de ramipril ; et déprotection de préférence par réduction avec un agent d'hydrogénation approprié. 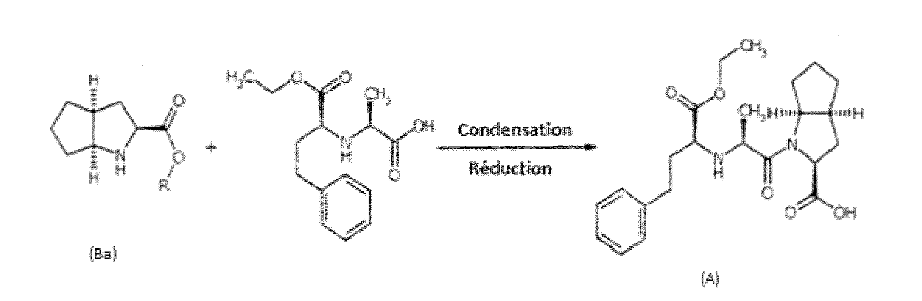 ou ;
ii) réaction de l'acide octahydrocyclopenta[b]pyrrole-2-carboxylique (IIa) intermédiaire, avec le composé de formule (D), pour donner le ramipril (A)

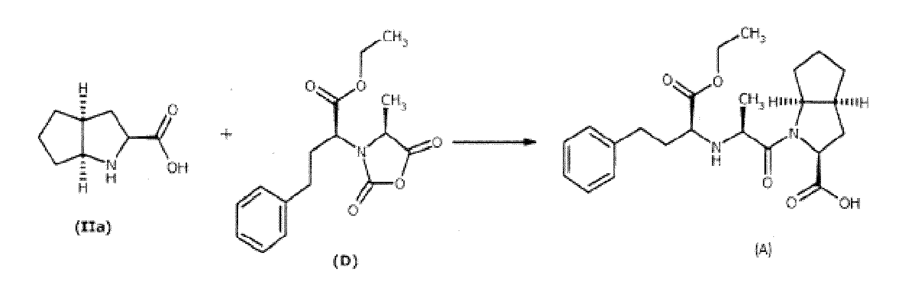

20. Procédé de préparation du ramipril, comprenant :
(A) la synthèse énantiospécifique de l'acide (2S)-acétylamino-3-(2-oxocyclopentyl)-propionique optiquement pur de formule (I), 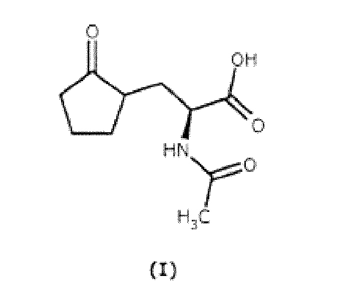 l'hydrolyse d'un mélange stéréoisomérique d'un propanoate d'(alkyle en C1-4)-2-(acétylamino)-3-(2-oxocyclopentyle) de formule (II), dans laquelle R est un alkyle (en C1-4), 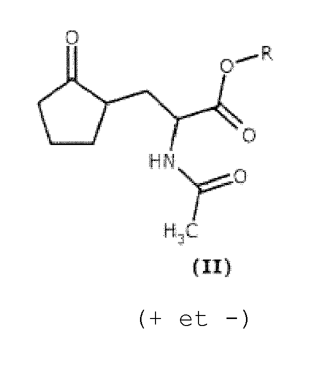 avec une sérine endopeptidase alcaline, pour former un mélange de l'isomère (+) de l'acide (2S)-acétylamino-3-(2-oxocyclopentyl)-propionique de formule (I) et de l'isomère (-) du propanoate d'(alkyle en C1-4) (2R)-2-(acétylamino)-3-(2-oxocyclopentyle) de formule (III) 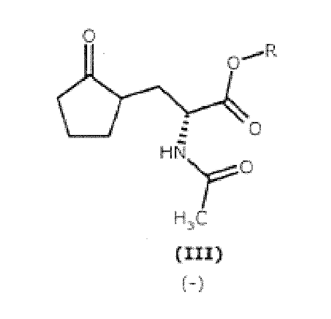
B) séparation de l'isomère (-) (III) non souhaité de l'isomère (I) optiquement pur par extraction avec un solvant organique choisi parmi le dichlorométhane, l'acétate d'éthyle et le trichlorométhane ;
(C) récupération et racémisation de l'isomère (III) non souhaité en le mélange stéréoisomérique (II), par
i. concentration de la couche organique ;
ii. traitement du résidu avec un alcool approprié et mise au reflux du mélange ;
iii. traitement de la masse réactionnelle avec un alcoxyde d'alcali approprié ;
iv. chauffage du mélange au reflux et refroidissement progressif du mélange ; et
v. agitation de la masse réactionnelle et isolement du composé racémique (II) (+ et -) par distillation ;
vi. recyclage du composé racémique (II) (+ et -) obtenu à l'étape (v) vers l'étape (A)
(D) cyclisation hydrolytique de l'intermédiaire (I) optiquement pur obtenu à l'étape (A) par mise au reflux de (I) en présence d'un agent de cyclisation approprié ; 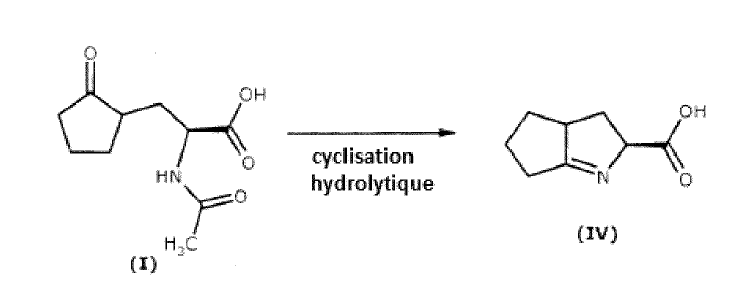
i. réduction pour obtenir l'acide (C) ; et facultativement
ii. estérification pour obtenir (Ba) optiquement pur 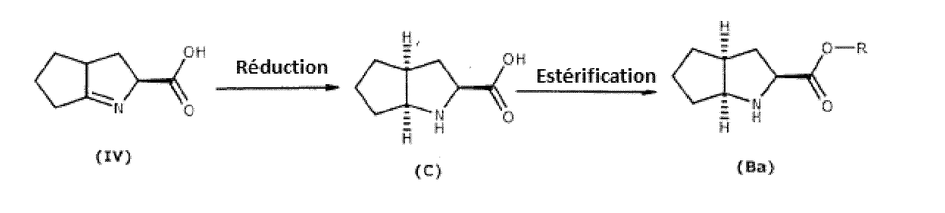
(E) condensation de l'ester de formule (Ba) formé à l'étape (C), avec de l'acide (2S)-2-([(2S)-1-éthoxy-1-oxo-4-phénylbutan-2-yl]amino)propanoïque pour former un ester de ramipril ; et déprotection de préférence par réduction avec un agent d'hydrogénation approprié ; 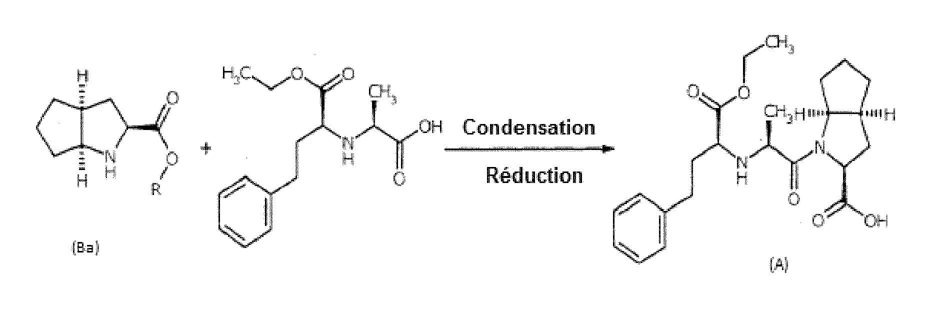 ou ;
réaction de l'acide octahydro-cyclopenta[b]pyrrole-2-carboxylique (IIa) intermédiaire, avec un composé de formule (D), pour donner du ramipril (A) :
